(19) Europäisches Patentamt
European Patent Office
Office européen des brevets

(11) Veröffentlichungsnummer: **0 227 057 B1**

# (12) EUROPÄISCHE PATENTSCHRIFT

(45) Veröffentlichungstag der Patentschrift: **21.08.91**

(51) Int. Cl.[5]: **C07C 29/34**, C07C 33/20, C07D 213/30

(21) Anmeldenummer: **86117718.6**

(22) Anmeldetag: **19.12.86**

## (54) Verfahren zur Herstellung von in 1-Stellung durch aromatische oder heterocyclische Reste substituierten Alkanolen.

(30) Priorität: **24.12.85 DE 3546016**

(43) Veröffentlichungstag der Anmeldung: **01.07.87 Patentblatt 87/27**

(45) Bekanntmachung des Hinweises auf die Patenterteilung: **21.08.91 Patentblatt 91/34**

(84) Benannte Vertragsstaaten: **BE CH DE FR GB IT LI NL SE**

(56) Entgegenhaltungen:

**HOUBEN-WEYL: "Methoden der Organischen chemie", Band VI/1b, Alkohole Teil III, Seiten 647-652, Georg Thieme Verlag, Stuttgart, DE**

(73) Patentinhaber: **BASF Aktiengesellschaft**
**Carl-Bosch-Strasse 38**
**W-6700 Ludwigshafen(DE)**

(72) Erfinder: **Bott, Kaspar, Dr.**
**Rieslingweg 4**
**W-6706 Wachenheim(DE)**
Erfinder: **Hoffmann, Herwig, Dr.**
**Knietschstrasse 21**
**W-6710 Frankenthal(DE)**
Erfinder: **Scheidmeir, Walter, Dr.**
**Hardenburgstrasse 41**
**W-6703 Limburgerhof(DE)**
Erfinder: **Trapp, Horst, Dr.**
**Johann-Sebastian-Bach-Strasse 10a**
**W-6831 Plankstadt(DE)**

**EP 0 227 057 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Diese Erfindung betrifft ein Verfahren zur Herstellung von Alkanolen, die in 1-Stellung durch aromatische oder heterocyclische Reste substituiert sind, durch Umsetzung sekundärer Alkohole mit primären Alkoholen.

Bekanntlich erhält man aus aliphatischen Alkoholen unter der Einwirkung von Alkalihydroxiden bei höheren Temperaturen durch Eigenkondensation höher molekulare Isoalkohole. Diese nach Guerbet benannte Reaktion (s. Angew. Chem. 64. Jahrgang 1952, S. 213-220) läuft nach dem Formelschema ab:

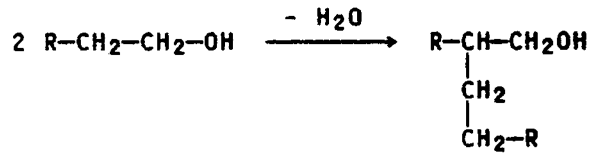


IV V

wobei R ein Alkylrest bedeutet.

Auf diese Weise läßt sich z.B. aus n-Butanol in hohen Ausbeuten 2-Ethylhexanol (R = $C_2H_5$) gewinnen, wenn man das bei der Kondensation gebildete Wasser aus der Reaktionsmischung abdestilliert. Die gute Selektivität einer solchen Guerbet-Synthese wird vor allem deshalb erreicht, weil die gemischte Kondensation des verzweigten Alkohols der Formel V mit dem Einsatzstoff der Formel IV eine wenig begünstigte Reaktion darstellt.

Später hat man festgestellt, daß man nach diesem Reaktionsprinzip auch "gekreuzte" Guerbet-Synthesen mit hoher Selektivität durchführen kann, wenn man außer dem Alkanol als weitere Reaktionskomponente Methanol (Bulletin Soc. Chim. France 1983, II. S. 252-256) oder Benzylalkohol (Tetrahedron, 1967, Vol. 23, S. 1723-1733) einsetzt. Im letztgenannten Fall tritt die Hydroxylgruppe des Benzylalkohols im Reaktionswasser auf, während sich der HO-Rest des Alkanols in dem Kondensationsprodukt wiederfindet:

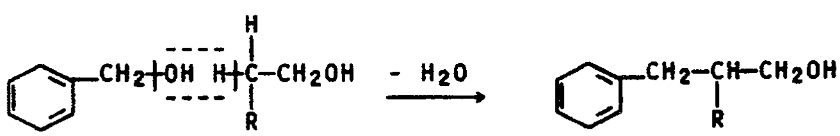


Es wurde nun überraschenderweise gefunden, daß man Alkanole der Formel

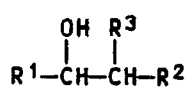

I,

in der $R^1$ einen aromatischen oder heterocyclischen Rest, $R^2$ einen linearen oder verzweigten Alkylrest mit 1 bis 20 C-Atomen und $R^3$ ein Wasserstoffatom, Methyl oder Ethyl bedeuten erhält, wenn man einen sekundären Alkohol der Formel

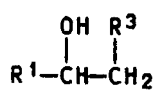

II

mit einem primären Alkohol der Formel $R^2OH$ (III), wobei die Reste $R^1$, $R^2$ und $R^3$ die oben genannte Bedeutung haben, in Gegenwart eines Alkalihydroxids bei Temperaturen von 150 bis 300°C umsetzt.

Das neue Verfahren gestattet es, auf besonders wirtschaftliche Weise, die in 1-Stellung durch aromatische oder heterocyclische Reste substituierten Alkanole herzustellen. Überraschenderweise ist die Hydroxylgruppe in den Ausgangsstoffen der Formel II nicht an der chemischen Umsetzung beteiligt. Es konnte auch nicht erwartet werden, daß sich als Alkanole der Formel III solche mit verzweigten Kohlenstoffketten

mit so gutem Erfolg einsetzen lassen, und daß z.B. beim Einsatz von Alkanolen der Formel IV deren Eigenkondensation zu den Verbindungen der Formel V weitgehend unterbleibt.

In den sekundären Alkoholen der Formel II steht $R^1$ für einen aromatischen Rest, wie für einen Phenylrest, der noch Substituenten, wie Alkylgruppen mit 1 bis 4 C-Atomen oder Phenylreste enthalten kann, oder für einen heterocyclischen Rest, wie einen Pyridylrest oder einen Chinolylrest. Im einzelnen sind als Alkohole der Formel II genannt: 1-Phenylethanol, 1-Phenylpropanol, 1-Phenylbutanol, 1-(3-Pyridyl)-ethanol, 1-(3-Pyridyl)-propanol, 1-(3-Pyridyl)-butanol, 1-(2-Chinolyl)-ethanol, 1-(3-Chinolyl)-ethanol, 1-(3-Chinolyl)-propanol.

Die primären Alkohole der Formel III sind geradkettige oder verzweigte Alkanole mit Alkylresten, die 1 bis 20, vorzugsweise 1 bis 15 C-Atome aufweisen. Im einzelnen seien z.B. genannt: n-Propanal, n-Butanal, 2-Ethylhexanol und die durch Hydroformylierung von "Dimer-Buten" bzw. "Trimer-Propylen" leicht zugänglichen iso-Nonanole bzw. iso-Dekanole.

Man setzt die sekundären Alkohole der Formel II den primären Alkoholen der Formel III in Gegenwart eines Alkalihydroxids, wie Kalium- oder Natriumhydroxid bei Temperaturen von 150 bis 340 °C, bevorzugt bei 200 bis 300 °C, insbesondere bei 200 bis 270 °C um. Das Mischungsverhältnis zwischen den Alkoholkomponenten kann in weiten Grenzen, z.B. von 1 Mol II zu 5 Mol III bis 5 Mol II zu 1 Mol III, schwanken. Bevorzugt werden die Ausgangsstoffe im molaren Verhältnis eingesetzt. Das Alkalihydroxid wird in Mengen von 0,5 bis 5, bevorzugt 0,5 bis 3, insbesondere 0,5 bis 1,5 Gew.%, bezogen auf das Gemisch der beiden Alkohole angewendet. Um einen möglichst hohen Umsatz der Reaktanden II und III zu erreichen, ist es vorteilhaft, das gebildete Wasser mit einem der Ausgangsstoffe abzudestillieren.

Der Nutzen des erfindungsgemäßen Verfahrens gewinnt dadurch an Bedeutung, daß man die auf diesem Wege zugänglichen Alkohole durch katalytische Hydrierung in die entsprechenden Kohlenwasserstoffe umwandeln und somit lineare oder verzweigte Kohlenstoffketten definierter Struktur in einen aromatischen Ring einführen kann. Weiterhin lassen sich die Verfahrensprodukte durch Dehydrierung der sekundären HO-Gruppe in die korrespondierenden Acylophenone überführen, die man normalerweise auf kompliziertere Weise durch Umsetzung von Carbonsäurechloriden mit einem aromatischen Kohlenwasserstoff unter Einsatz von äquimolaren Mengen Aluminiumchlorid herstellt.

Beispiele 1 bis 4

In einem Rührautoklaven mit einem Inhalt von 0,3 l wurden äquimolare Teile der in der Tabelle als Einsatzstoffe genannten Alkohole der Formeln II und III zusammen mit 1 Gew.%, bezogen auf das Alkoholgemisch, Kaliumhydroxid 10 Stunden bei 270 °C umgesetzt.

Auf die Ausschleusung des Reaktionswassers während der Umsetzung wurde verzichtet. Aus den gaschromatographischen Analysen der Reaktorausträge wurden sowohl der Umsatz als auch die Selektivität der Bildung der Alkanole der Formel I berechnet, indem man für alle Komponenten im Gaschromatogramm die Flächenprozente mit den Gewichtsprozenten gleichsetzte.

Die Formeln der entstandenen Alkanole, der Umsatz und die Selektivität sind der Tabelle zu entnehmen.

Tabelle

| Beispiel Nr. | Einsatzstoffe II | Einsatzstoffe III | Strukturformel des gebildeten Alkohols der Formel I | Selektivität, bezogen auf umgesetzten Alkohol II | Umsatz, bezogen Alkohol II |
|---|---|---|---|---|---|
| 1 | 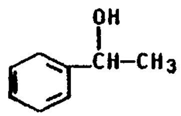 | $n-C_4H_9-OH$ | 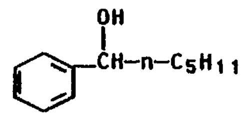 | 86 % | 64 % |
| 2 | 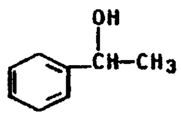 | 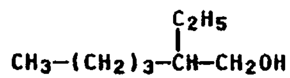 | 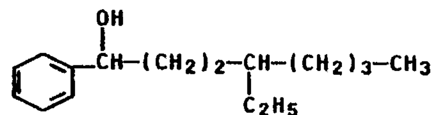 | 90 % | 62 % |
| 3 | 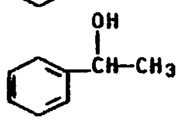 | $iso-C_9H_{19}-OH$ | 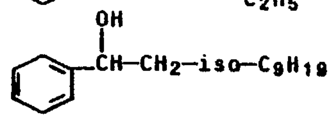 | 95 % | 62 % |
| 4 | 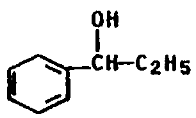 | $CH_3OH$ | 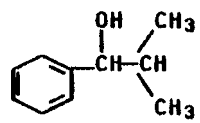 | 98 % | 34 % |

## Patentansprüche

1. Verfahren zur Herstellung von Alkanolen der Formel

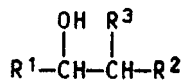

I,

in der $R^1$ einen aromatischen oder heterocyclischen Rest, $R^2$ einen linearen oder verzweigten Alkylrest mit 1 bis 20 C-Atomen und $R^3$ ein Wasserstoffatom, Methyl oder Ethyl bedeuten, dadurch gekennzeichnet, daß man einen sekundären Alkohol der Formel

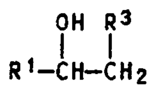

II

mit einem primären Alkohol der Formel $R^2OH$ (III), wobei die Reste $R^1$, $R^2$ und $R^3$ die oben genannte Bedeutung haben, in Gegenwart eines Alkalihydroxids bei Temperaturen von 150 bis 300 °C umsetzt.

2. Verfahren nach Anspruch 1, dadurch gekennzeichnet, daß man als Alkalihydroxid Kaliumhydroxid verwendet.

3. Verfahren nach Anspruch 1, dadurch gekennzeichnet, daß man bei Temperaturen von 200 bis 300 °C umsetzt.

4. Verfahren nach Anspruch 1, dadurch gekennzeichnet, daß der sekundäre Alkohol der Formel II als aromatischen Rest $R^1$ einen Phenylrest enthält, der noch Alkylgruppen mit 1 bis 4 C-Atomen enthalten kann.

5. Verfahren nach Anspruch 1, dadurch gekennzeichnet, daß der sekundäre Alkohol der Formel II als heterocyclischen Rest $R^1$ einen Pyridylrest enthält.

6. Verfahren nach Anspruch 1, dadurch gekennzeichnet, daß man die Ausgangsstoffe im Mischungsverhältnis 1 Mol II zu 5 Mol III bis 5 Mol II zu 1 Mol III einsetzt.

7. Verfahren nach Anspruch 1, dadurch gekennzeichnet, daß man das Alkalihydroxid in Mengen von 0,5 bis 5 Gew.%, bezogen auf das Gemisch der beiden Alkohole, anwendet.

**Claims**

1. A process for the preparation of an alkanol of the formula

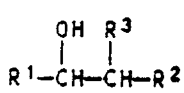

I

where $R^1$ is an aromatic or heterocyclic radical, $R^2$ is a straight-chain or branched alkyl radical of 1 to 20 carbon atoms and $R^3$ is hydrogen, methyl or ethyl, wherein a secondary alcohol of the formula

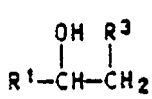

II

is reacted with a primary alcohol of the formula $R^2OH$ (III) where $R^1$, $R^2$ and $R^3$ have the above meanings, in the presence of an alkali metal hydroxide at from 150 to 300 °C.

2. A process as claimed in claim 1, wherein potassium hydroxide is used as the alkali metal hydroxide.

**3.** A process as claimed in claim 1, wherein the reaction is carried out at from 200 to 300 °C.

**4.** A process as claimed in claim 1, wherein the secondary alcohol of the formula II contains, as the aromatic radical $R^1$, phenyl which may furthermore contain alkyl groups of 1 to 4 carbon atoms.

**5.** A process as claimed in claim 1, wherein the secondary alcohol of the formula II contains pyridyl as the heterocyclic radical $R^1$.

**6.** A process as claimed in claim 1, wherein the molar ratio of the starting material II to starting material III in the mixture is from 1:5 to 5:1.

**7.** A process as claimed in claim 1, wherein the alkali metal hydroxide is used in an amount of from 0.5 to 5% by weight, based on the mixture of the two alcohols.

## Revendications

**1.** Procédé de préparation d'alcanols de formule

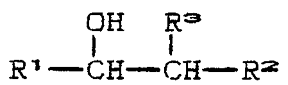

I,

dans laquelle $R^1$ désigne un reste aromatique ou hétérocyclique, $R^2$ désigné un reste alkyle linéaire ou ramifié contenant 1 à 20 atomes de carbone et $R^3$ désigne un atome d'hydrogène, un radical méthyle ou éthyle, caractérisé en ce que l'on fait réagir un alcool secondaire de formule

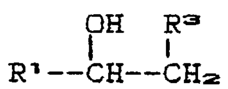

II

avec un alcool primaire de formule $R^2OH$ (III), les restes $R^1$, $R^2$ et $R^3$ ayant les significations susindiquées, en présence d'un hydroxyde alcalin, à des températures de 150 à 300 °C.

**2.** Procédé suivant la revendication 1, caractérisé en ce que l'on utilise comme hydroxyde alcalin l'hydroxyde de potassium.

**3.** Procédé suivant la revendication 1, caractérisé en ce que l'on effectue la réaction à des températures de 200 à 300 °C.

**4.** Procédé suivant la revendication 1, caractérisé en ce que l'alcool secondaire de formule II contient un reste phényle en tant que reste aromatique $R^1$, ce reste phényle pouvant encore contenir des groupes alkyle ayant 1 à 4 atomes de carbone.

**5.** Procédé suivant la revendication 1, caractérisé en ce que l'alcool secondaire de formule II contient un reste pyridyle en tant que reste hétérocyclique $R^1$.

**6.** Procédé suivant la revendication 1, caractérisé en ce que l'on met en oeuvre les substances de départ dans le rapport de mélange de 1 mole de II pour 5 moles de III à 5 moles de II pour 1 mole de III.

**7.** Procédé suivant la revendication 1, caractérisé en ce que l'on utilise l'hydroxyde alcalin en des quantités de 0,5 à 5% en poids par rapport au mélange des deux alcools.